# EUROPEAN PATENT APPLICATION

(11) **EP 3 031 449 A1**
(43) Date of publication of application: **15.06.2016**
(21) Application number: 13891194.6
(22) Date of filing: 06.08.2013
(51) Int. Cl.: A61K 9/16, A61K 47/48, A61K 47/30, A61K 31/52

(54) **ENTECAVIR MICROSPHERES AND PHARMACEUTICAL COMPOSITION FOR PARENTERAL ADMINISTRATION CONTAINING SAME**

(71) Applicant: Dong Kook Pharm. Co., Ltd, Gangnam-gu, Seoul 135-502 (KR)
(72) Inventor: EOM, Shin, Suwon-si Gyeonggi-do 441-884 (KR); LEE, Deok Keun, Seoul 156-090 (KR); CHA, Kyung Hoi, Yongin-si Gyeonggi-do 446-754 (KR)
(74) Representative: Schrell, Andreas
(86) International application number: PCT/KR2013/007085
(87) International publication number: WO 2015/020240

(57) **Abstract**

The present invention relates to entecavir microspheres and a pharmaceutical composition for parenteral administration containing the same. The entecavir microspheres are manufactured by a method comprising the steps of: dissolving entecavir and a biodegradable biocompatible polymer in at least one solvent; putting the solution of the entecavir and biodegradable biocompatible polymer in a hydrophilic polymer solution, followed by stirring, to form microspheres; and removing the solvent. The entecavir is sufficiently sealed in the entecavir microsphere at approximately 80% or more based on the input amount, and the elution of the entecavir is maintained for 30 days or more, and thus the entecavir microspheres can continuously exhibit efficacy, thereby improving medication compliance of patients.

## Description

### TECHNICAL FIELD

The present invention relates to a microsphere of entecavir and an injectable composition comprising the same. The present invention provides entecavir-which has been conventionally used in oral administration only-as a sustained-release pharmaceutical composition for parenteral administration by preparing a microsphere of a biodegradable, biocompatible polymer.

### BACKGROUND ART

Entecavir-which is a guanosine nucleoside analogue of the following chemical formula-has been used as a therapeutic agent for chronic hepatitis B virus (HBV) infection.

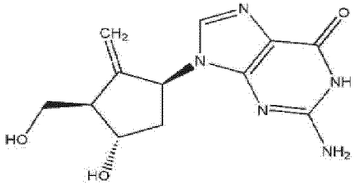

As a therapeutic agent for chronic HBV infection, entecavir should be taken every day for life. When orally administered, entecavir shows bioavailability which is nearly 100%. However, because the absorbance rate is decreased by the intake of food, it is inconvenient that entecavir should be orally taken 2 hours before a meal.

As to the prescription pattern of currently released oral preparations of entecavir, it is usual that doctors make prescription every about 1-33 months during the initial 1-2 years and make prescription every 6 months after stabilization of HBV. In the case of first-time patients treated in such a manner, after 1 year of administration about 70% of patients are HBV-undetectable, and after 3 years about 90% of patients are HBV-undetectable. As such, in the treatment of HBV infection long-term use of entecavir is necessary. There is a disadvantage of lowering drug compliance due to everyday oral administration, so that development of sustained-release preparations is necessary. However, up to now there is no case in which entecavir has been developed as a sustained-release microsphere.

Meanwhile, sustained-release injectable formulation (hereinafter referred to as "depot") refers to an injectable formulation in which drugs are formulated to be constantly and uniformly released in the body while maintaining biological activity at the time of subcutaneous or intramuscular injection. Aliphatic polyester-which has been developed and used as a polymer carrier of such a depot formulation-has already been recognized for its biocompatibility and approved by the US Food and Drug Administration (FDA), and has been widely used as a drug delivery carrier or a suture. Concrete examples of aliphatic polyester include poly-L-lactic acid, polyglycolic acid, poly-D-lactic acid-co-glycolic acid, poly-L-lactic acid-co-glycolic acid, poly-D,L-lactic acid-co-glycolic acid (hereinafter referred to as "PLGA"), polycaprolactone, poly-valerolactone, polyhydroxybutyrate and poly-hydroxyvalerate (Peppas, L. B., Int. J. Pharm., 116, 1-9, 1995).

As a method of preparing such a depot formulation, in general a phase separation method, a spray drying method, a solvent extraction method, a solvent evaporation method and the like have been known.

US Patent No. 4,673,595 discloses a depot formulation prepared by a phase separation method. However, there are disadvantages in that silicone oil, heptane and ethyl alcohol as well as methylene chloride solvent are used, and the procedure is complicated such as removal of all solvents used in the operation.

Korean Patent No. 0566573 discloses a spray drying method. However, there are disadvantages in that spray drying at high temperature of 60°C or more may cause damage to an active ingredient or drug delivery carrier, and if drying is carried out at low temperature, organic solvents used therein cannot be removed. In addition, Korean Patent No. 0537713 discloses a method in which an active ingredient is encapsulated into cholesterol by the use of a spray drying method, but various release time-retarding effects cannot be expected due to limited molecular weight kinds of cholesterol used therein and the interaction with an active ingredient, and its applications may be limited.

US Patent No. 5,366,734 discloses a continuous-release pharmaceutical composition in implant form in which a drug is mixed with a biodegradable polymer. However, the needle size for subcutaneous injection is enlarged due to the large size of the implant, so that there is a disadvantage in that drug compliance is lowered.

Korean Patent No. 0432677 discloses a method in which an active ingredient is in a sustained-release formulation by the use of a solvent extraction method. However, because the boiling point of the solvent used therein is very high, a long time is needed to extract and wash the solvent, thereby increasing the manufacturing cost. In addition, even after long-term extraction, the solvent is not completely removed. As a result, there is a disadvantage of lowering the stability of the product due to residual solvent.

Korean Patent Application Publication No. 2002-0011975 also discloses a method for preparing a microsphere by the use of a solvent extraction method. However, in this method the boiling point of the solvent used therein is also high. Therefore, there is a disadvantage in that even after extraction a considerable amount of the solvent remains in microspheres after washing.

US Patent No. 4,652,441 discloses a method for preparing a microcapsule in which after forming three-phase emulsions an active ingredient is encapsulated into a polymer by a drying-in-water method. However, in the case of a water-soluble active ingredient, when it is exposed to the external aqueous phase, the loading rate is decreased. Therefore, there is a problem in obtaining a microcapsule containing high drug content.

Korean Patent No. 0994658 discloses a method in which a microsphere of water-soluble peptide drug is produced by a multiple emulsion method of a solvent evaporation method. However, this method is applicable to an active ingredient having high water solubility only and not an active ingredient having low water solubility.

Up to now, examples of drugs developed as a sustained-release injection are those should be administered as an injection every day (gonadotropin-releasing hormone [GnRH] agonists: triptorelin, leuprolide, goserelin and the like) and those for psychoneurotic disorders (risperidone, olanzapine, paliperidone, naltrexone and the like). Considering that in the case of GnRH agonists, the inconvenience of patients having an injection every day is removed, and in the case of drugs for psychoneurotic disorders the problem of patients being unwilling to take a medicine, a formulation in which medicinal effect is maintained over a long period with one administration is needed in the clinical area. Accordingly, sustained-release injections have been developed. That is, other than specific cases such as drugs for psychoneurotic disorders, there is no need to develop oral preparations as injection preparations. In the case of entecavir, because it was developed as an oral preparation which can be easily administered, there is no need to develop an injection preparation. Still more, it is not easily conceivable to develop a sustained-release formulation by the use of a microsphere. As such, up to now entecavir has been administered via the oral route only.

In addition, in conventional methods of preparing a microsphere it is usual that the main ingredient(s) and polymer(s) are dissolved in a solvent such as methylene chloride, benzyl alcohol, ethyl acetate and the like as an organic phase, and this organic phase is added to an aqueous phase to obtain microspheres. However, because the solubility of entecavir with such organic solvent is not high, it is difficult to prepare microspheres.

In the present invention, entecavir-in which conception of a sustained-release injection formulation itself is not easy and there is a technical difficulty in preparing microspheres-has been developed as a sustained-release injection formulation to improve drug compliance. To this end, the inventors repeatedly conducted studies including the selection of solvents having sufficiently high solubility of entecavir instead of those conventionally used in the preparation of microspheres, and completed the present invention.

### DISCLOSURE OF INVENTION

### TECHNICAL PROBLEM

The present invention is intended to improve drug compliance of entecavir which has been administered via the oral route only. The object of the present invention is the provision of entecavir as a sustained-release pharmaceutical composition for parenteral administration by preparing a microsphere of a biodegradable, biocompatible polymer.

### SOLUTION TO PROBLEM

To accomplish the above object, the present invention provides an entecavir microsphere comprising entecavir or a pharmaceutically acceptable salt thereof, and a biodegradable, biocompatible polymer.

A method for preparing an entecavir microsphere according to the present invention comprising the steps of:
dissolving entecavir and at least one biodegradable, biocompatible polymer in at least one solvent;
forming a microsphere by adding the solution of entecavir and the biodegradable, biocompatible polymer to a hydrophilic polymer aqueous solution and agitating; and removing the solvent.

In the present invention, the biodegradable, biocompatible polymer is preferably selected from poly(lactide-co-glycolide), polylactide, polyglycolide, poly(lactide-co-glycolide)glucose, polycaprolactone, gelatin and hyaluronate, and more preferably polyglycolide, polylactide, and a copolymer of polyglycolide and polylactide. Specifically, in the case of poly(lactide-co-glycolide) copolymer, the molar ratio of lactide to glycolide is preferably 50 : 50 to 90 : 10.

The biodegradable, biocompatible polymer used in the present invention may have intrinsic viscosity of 0.1 to 1.9 dL/g, and preferably at least one polymer having intrinsic viscosity of 0.3 to 1.9 dL/g is used.

The entecavir microsphere according to the present invention may comprise 1 to 90% by weight of entecavir, and preferably 10 to 60% by weight of entecavir.

In addition, the size of the entecavir microsphere may be 1 to 250 µm, and preferably 10 to 200 µm.

The entecavir microsphere prepared according to the present invention is a sustained-release formulation, and release of entecavir may continue for 30 days or more.

Hereinafter, the present invention is explained in more detail.

In the conventional method for preparing microspheres, a drug and a polymer are dissolved in organic solvent such as methylene chloride and ethyl acetate, and this organic phase is added to an aqueous medium to form microspheres. However, in the case of entecavir, because it is insoluble with methylene chloride, such a method cannot be used. In the present invention, entecavir is dissolved in a solvent in which entecavir is well dissolved, and this solution is mixed with a solution in which a polymer is dissolved to obtain a dispersion phase. In the case of preparing microspheres according to the conventional method, the loading rate is low. Contrary to this, it was confirmed that when microspheres are prepared according to the present invention, the loading rate is excellent.

In the present invention, the solvent available for dissolving entecavir may include dimethyl sulfoxide, dimethylformamide, acetic acid, hydrochloric acid, methanol, ethanol, water for injection and the like. Specifically, dimethyl sulfoxide, dimethylformamide or acidic solution such as acetic acid and hydrochloric acid may be preferable.

In addition, the solvent available for dissolving a polymer may include methylene chloride, acetone, chloroform, acetonitrile, ethyl acetate, dimethyl sulfoxide, dimethylformamide and the like.

In the hydrophilic polymer solution in which the dispersion solution of entecavir solution and the polymer solution is added thereto to obtain microspheres, a polymer such as polyvinyl alcohol, polyvinylpyrrolidone, hydroxypropyl cellulose, hydroxypropyl methylcellulose and the like may be used.

In the preparation of an entecavir microsphere according to the present invention, the removal of the organic solvent may be carried out by applying any of the solvent removal methods conventionally used in this field-for example, agitation, heating, N₂ purge and the like.

In addition, the present invention provides a pharmaceutical composition for parenteral administration comprising the entecavir microsphere prepared as above and a pharmaceutically acceptable carrier. Specifically, the pharmaceutical composition for parenteral administration is preferably an injection.

The pharmaceutical composition comprising the entecavir microsphere according to the present invention can be used as a therapeutic agent for chronic hepatitis B virus infection. The administration dose may be variously adjusted according to the age of patients, symptoms, dosage form and the like. Entecavir may be administered at a dose of 0.1 to 5.0 mg/day, preferably 0.2 to 3.0 mg/day, and more preferably 0.5 to 1.0 mg/day.

In addition, the pharmaceutical composition according to the present invention may be prepared by the use of a conventional pharmaceutically acceptable carrier, and conventional preparation methods may be used to obtain high bioavailability.

The entecavir microsphere of the present invention may be provided as a preparation in which the entecavir microsphere is suspended in an injectable suspending agent at the time of final administration. The injectable suspending agent is a water-soluble organic carrier, and its example includes, but is not limited to, an isotonic agent, a thickening agent, a surfactant and a buffer. Examples of available isotonic agent are, but are not limited to, a water-soluble excipient such as mannitol, sucrose, sorbitol, trehalose, lactose, or sugars. Examples of thickening agent are, but are not limited to, carmellose sodium, sodium carboxymethyl cellulose or povidone. Examples of surfactant are, but are not limited to, polyoxyethylene sorbitans such as Polysorbate 80 or Polysorbate 20, or sorbitan esters such as Span 80 or Span 20. In addition, examples of buffer are, but are not limited to, sodium monohydrogen phosphate, anhydrous citric acid, sodium hydroxide or sodium chloride.

### EFFECTS OF INVENTION

In the entecavir microsphere prepared according to the present invention, an average of 80% or more of entecavir based on the amount of input is sufficiently loaded into the microsphere, and release of entecavir continues for 30 days or more. As a result, in the case of preparing as a parenteral preparation such as injection, it is safe and shows continuous effect, thereby improving drug compliance of patients.

### BRIEF DESCRIPTION OF DRAWINGS

Figure 1 is photographs showing SEM analysis results for microspheres prepared in the Example and Comparative Example (Figure 1a: microspheres of Example 6, Figure 1b: microspheres of the Comparative Example).
Figure 2 represents the particle size distribution of microspheres prepared in Example 4.
Figures 3 and 4 are graphs representing the dissolution rate (%) of microspheres prepared in the Examples and the Comparative Example depending on time in the results of *in vitro* long-term dissolution test.
Figure 5 is microscopic photographs of injection sites of rats taken 4 weeks after subcutaneous administration of entecavir and excipient (Figure 5a: 2.33 mg/kg/day of entecavir-administration group, Figure 5b: excipient-administration group).

### MODE FOR THE INVENTION

Hereinafter, the present invention is explained in more detail with the following examples. However, the following examples are only intended to facilitate understanding of the present invention, and the protection scope of the present invention is not limited thereto.

### Examples 1 to 3: Preparation of microspheres

2.5 g of entecavir (EstechPharma Co., Ltd.) was dissolved in 40.0 g of dimethyl sulfoxide (first organic phase), and 7.5 g of biodegradable polymers (Boehringer Ingelheim) represented in Table 1 were dissolved in 27.0 g of methylene chloride (Merck) (second organic phase). The completely dissolved first and second organic phases were sufficiently mixed to obtain a dispersion solution. This dispersion solution was slowly added to 0.5% polyvinyl alcohol (Mn = 30,000-70,000; Sigma) aqueous solution at 20°C, and microspheres were prepared by the use of an L4RT mixer (Siverson, UK) at 3,000 rpm. Next, the temperature was increased to 47°C in order to volatilize organic solvents and then cooled to 20°C for the filtration of microspheres. The filtered microspheres were washed with water for injection several times and freeze-dried to obtain microspheres.

The biodegradable polymers used in these Examples are represented in Table 1.

**[Table 1]**

| Example | Polymer |
|---|---|
| 1 | RG502H |
| 2 | RG756S : RG502H (8 : 2) |
| 3 | RG756S : RG504H (8 : 2) |

All polymers used in the above Examples are copolymers; RG means polylactide-glycolide copolymer, H means hydrophilic end-group, and S means hydrophobic end-group. In the middle number, the first two digits mean the ratio of lactide in copolymer. For example, 50 means a copolymer in which lactide is 50%, and 75 means a copolymer in which lactide is 75%. And the last digit of the number means the grade of polymer and the difference of intrinsic viscosity. For example, 502H means that intrinsic viscosity is 0.16-0.24 dL/g, and 504H means 0.45-0.60 dL/g.

### Example 4: Preparation of microspheres

2.5 g of entecavir (EstechPharma Co., Ltd.) and 7.5 g of biodegradable polymer (RG756S, Boehringer Ingelheim) were weighed, and 40.0 g of dimethyl sulfoxide and 27.0 g of methylene chloride were added thereto, respectively, to dissolve them (first organic phase). Each organic phase was sufficiently mixed, and the dissolved organic phases were slowly added to 0.5% polyvinyl alcohol (Mn = 30,000-70,000; Sigma) aqueous solution at 20°C, and microspheres were prepared by the use of an L4RT mixer (Siverson, UK) at 3,000 rpm. Next, the temperature was increased to 47°C in order to volatilize organic solvents and then cooled to 20°C for the filtration of microspheres. The filtered microspheres were washed with water for injection several times and freeze-dried to obtain microspheres.

### Example 5: Preparation of microspheres

2.5 g of entecavir (EstechPharma Co., Ltd.) and 7.5 g of biodegradable polymer (RG756S : RG504H = 8 : 2, Boehringer Ingelheim) were weighed, and 30.0 g of acetic acid and 37.0 g of methylene chloride were added thereto, respectively, to dissolve them (first organic phase). Each organic phase was sufficiently mixed, and the dissolved organic phases were slowly added to 0.5% polyvinyl alcohol (Mn = 30,000-70,000; Sigma) aqueous solution at 20°C, and microspheres were prepared by the use of an L4RT mixer (Siverson, UK) at 3,000 rpm. Next, the temperature was increased to 47°C in order to volatilize organic solvents and then cooled to 20°C for the filtration of microspheres. The filtered microspheres were washed with water for injection several times and freeze-dried to obtain microspheres.

### Comparative Example

90.61 g of 1% polyvinyl alcohol, 2.97 g of benzyl alcohol and 6.53 g of ethyl acetate were mixed to obtain an aqueous phase. And 2.93 g of biodegradable polymer (RG757S) was dissolved in 10.87 g of ethyl acetate and 10.84 g of benzyl alcohol. After complete dissolution of the polymer, 1.57 g of entecavir was added thereto to obtain an organic phase. While injecting the obtained organic phase to the aqueous phase, microspheres were formed by the use of an L4RT mixer (Siverson, UK) at 3,000 rpm. Next, polyvinyl alcohol suspension in which microspheres are dispersed was added to water for injection at 10°C containing 127.6 g of ethyl acetate, 9.23 g of anhydrous sodium bicarbonate and 11.62 g of anhydrous sodium carbonate, and the organic solvents were extracted and microspheres were washed. After filtration of the obtained microspheres, the filtered microspheres were washed with water for injection several times and freeze-dried to obtain microspheres.

The Comparative Example is a modification of the example for preparing risperidone microspheres disclosed in Korean Patent No. 10-0354270 in which the main ingredient is changed to entecavir.

### Experimental Example 1: Observation of microsphere morphology

To observe the surface of the microspheres prepared in the Example and Comparative Example, about 10 mg of the microspheres were fixed on an aluminum stub and coated with platinum under 0.1 torr of degree of vacuum and high voltage (10 kV) for 3 minutes. The microspheres were installed on a scanning electron microscope (SEM) (Hitachi S-4800 FE-SEM), and then the surface of the microspheres was observed by using an image-analysis program.

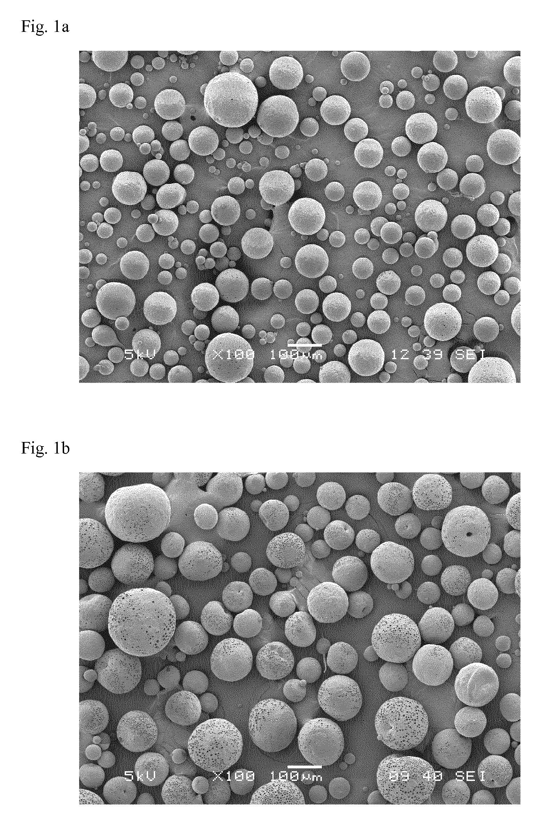

Figure 1 is photographs showing SEM analysis results for the morphology of the microspheres prepared in the Example and Comparative Example. Figure 1a is an SEM photograph of microspheres of Example 6, and Figure 1b is that of the Comparative Example.

As can be seen from Figure 1, the porosity of the surface in the microspheres of the Example is decreased as compared with that of the Comparative Example, in which the loading rate can be expected to increase. In addition, it can be known that regulation of drug release over various periods is easy by decreasing the surface area due to the decreased porosity.

### Experimental Example 2: Measurement of microsphere particle size distribution

To observe the particle size distribution of microspheres, the particle size was measured by a wet method. The measurement of particle size was carried out by the use of a HELOS/KFS (SYMPATEC) equipped with SUCELL disperser and R5 lens.

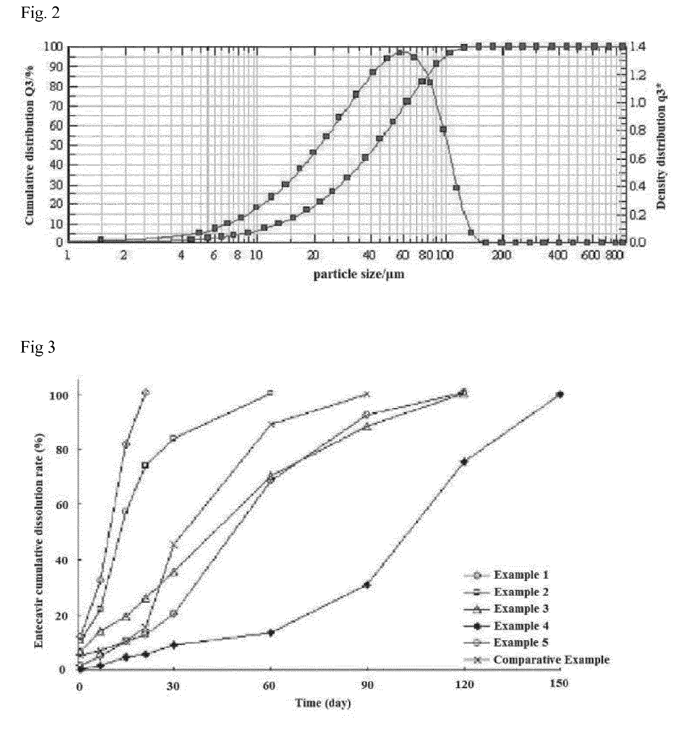

Figure 2 represents the particle size distribution of microspheres prepared in Example 4.

### Experimental Example 3: Measurement of loading rate of entecavir

About 50 mg of the microspheres was added to 20 mL of dimethyl sulfoxide in a 100 mL flask and completely dissolved, and the volume was then adjusted to the marked line with hydrochloric acid test solution (0.01 mol/L). This solution was filtrated with a 0.45 µm syringe filter to obtain a test solution. The content of entecavir loaded into the microspheres was measured by HPLC under the following conditions:
Column: YMC C18 ODS 5 µm, 4.6 × 150 mm
Loading amount: 100 µl
Detection wavelength: 254 nm
Mobile phase = water : acetonitrile (92 : 8).

The results are represented in Table 2.

**[Table 2]**

| | Amount of entecavir (%) | Drug loading rate (%) |
|---|---|---|
| Example 1 | 19.6 | 78.4 |
| Example 2 | 20.1 | 80.4 |
| Example 3 | 20.3 | 81.2 |
| Example 4 | 20.8 | 83.2 |
| Example 5 | 19.8 | 79.2 |
| Comparative Example | 10.1 | 40.4 |

As can be seen from Table 2, in all microspheres prepared according to the present invention about 80% or more of entecavir based on the input amount was sufficiently loaded into the microspheres. Contrary to this, in the method of the Comparative Example the drug loading rate was about 50% or less of that of the Examples.

### Experimental Example 4: In vitro long-term dissolution test of microspheres

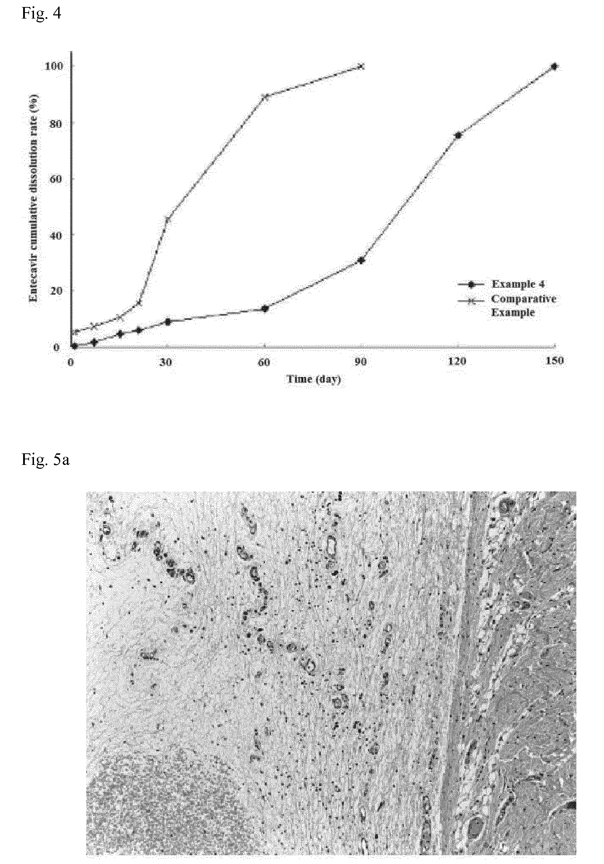

After placing about 20 mg of the microspheres prepared in Examples 1 to 5 and the Comparative Example into a 250 mL wide-mouth bottle, 200 mL of HEPES solution (pH 7.4) was added thereto and incubated at 37°C. Supernatant was taken at regular intervals for 1 to 5 months to measure the dissolution rate of entecavir by HPLC. The measured results and a graph thereof are represented in Table 3 and Figures 3 and 4.

**[Table 3]**

| | Day 1 | Day 7 | Day 15 | Day 21 | Day 30 | Day 60 | Day 90 | Day 120 | Day 150 |
|---|---|---|---|---|---|---|---|---|---|
| Example 1 | 12.1 | 32.7 | 81.9 | 100.5 | - | - | - | | |
| Example 2 | 10.4 | 22.2 | 57.3 | 74.1 | 84.1 | 100.1 | - | - | - |
| Example 3 | 6.8 | 14.2 | 19.4 | 26.3 | 35.8 | 70.2 | 88.3 | 100.2 | - |
| Example 4 | 0.3 | 1.6 | 4.8 | 5.9 | 9.1 | 13.5 | 30.8 | 75.5 | 99.9 |
| Example 5 | 1.6 | 5.2 | 10.6 | 13.0 | 20.5 | 68.6 | 92.3 | 100.5 | - |
| Comp. Ex. | 5.4 | 7.3 | 10.5 | 15.7 | 45.4 | 89.1 | 100.0 | - | - |

Figures 3 and 4 are graphs representing the dissolution rate (%) of microspheres prepared in the Examples and the Comparative Example depending on time in the results of the *in vitro* long-term dissolution test.

As can be seen from Table 3 and the Figures, it was confirmed that in entecavir microspheres prepared according to the present invention the release of the drug over 30 days can be controlled by altering the kind of biodegradable, biocompatible polymer. In addition, it can be known that the release of the drug was retarded when the ratio of lactide in polylactide-glycolide copolymer was high.

### Experimental Example 5: Histopathological examination for local toxicity of entecavir

A test was conducted to determine whether local irritation is caused by subcutaneous administration of entecavir into rats.

After entecavir (2.33 mg/kg/day) was subcutaneously injected into 15 male rats per each group, mortality, common symptoms, body weight, autopsy and histopathological evaluation on 1, 4, 5, 8 and 12 weeks were checked to compare with the control group in which excipient was administered.

As a result, no dead animal was observed in all groups, and abnormal symptoms and abnormal body weight change related to the administration of the test material also were not observed. From the autopsy results, there was no abnormal finding. In the entecavir-administration group, inflammation was observed. However, because all animals recovered from inflammation, it seems not to be a toxicologically significant change.

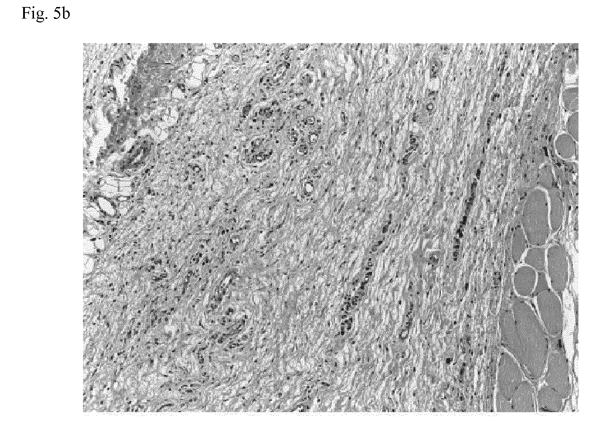

Figure 5 is microscopic photographs (H&E staining, × 100) of injection sites of rats taken 4 weeks after subcutaneous administration of entecavir and excipient. Figure 5a is a photograph of the entecavir (2.33 mg/kg/day)-administration group, and Figure 5b is a photograph of the excipient-administration group. In Figure 5a, some subcutaneous tissue inflammatory cells were observed. However, such inflammatory cells were also observed in the excipient-injection site, and thus it is not significant.

From the above results, it can be known that the group in which entecavir is subcutaneously administered did not show harmful local toxicity.

### Preparation Example: Preparation of Injection

Preparation of diluent: 230 mg of sodium carboxymethyl cellulose, 10 mg of polysorbate 20, 10 mg of monobasic sodium phosphate and 60 mg of sodium chloride were dissolved in water for injection to make 10 mL, and sodium hydroxide was then added thereto to adjust pH to 7.0.

At the time of administering to patients, 2 ml of the above diluent was added to a vial in which 270 mg of entecavir was charged and suspended for injection.

## Claims

1. An entecavir microsphere comprising entecavir or a pharmaceutically acceptable salt thereof, and a biodegradable, biocompatible polymer.

2. The entecavir microsphere according to Claim 1, wherein the biodegradable, biocompatible polymer is at least one of poly(lactide-co-glycolide), polylactide, polyglycolide, poly(lactide-co-glycolide)glucose, polycaprolactone, gelatin and hyaluronate.

3. The entecavir microsphere according to Claim 2, wherein the biodegradable, biocompatible polymer is at least one of polyglycolide, polylactide, and a copolymer of polyglycolide and polylactide.

4. The entecavir microsphere according to Claim 3, wherein the copolymer of polyglycolide and polylactide has the molar ratio of lactide to glycolide of 50 : 50 to 90 : 10.

5. The entecavir microsphere according to Claim 1, which comprises 10 to 60% by weight of entecavir.

6. The entecavir microsphere according to Claim 1, wherein the size of the microsphere is 10 to 200 µm.

7. The entecavir microsphere according to Claim 1, wherein release of entecavir continues for 30 days or more.

8. A method for preparing an entecavir microsphere comprising the steps of:
dissolving entecavir and at least one biodegradable, biocompatible polymer in at least one solvent;
forming a microsphere by adding the solution of entecavir and the biodegradable, biocompatible polymer to a hydrophilic polymer aqueous solution and agitating; and
removing the solvent.

9. The method according to Claim 8, wherein the biodegradable, biocompatible polymer has intrinsic viscosity of 0.1 to 1.9 dL/g.

10. The method according to Claim 8, wherein the biodegradable, biocompatible polymer is at least one of poly(lactide-co-glycolide), polylactide, polyglycolide, poly(lactide-co-glycolide)glucose, polycaprolactone, gelatin and hyaluronate.

11. The method according to Claim 10, wherein the biodegradable, biocompatible polymer is at least one of polyglycolide, polylactide, and a copolymer of polyglycolide and polylactide.

12. The method according to Claim 11, wherein the copolymer of polyglycolide and polylactide has the molar ratio of lactide to glycolide of 50 : 50 to 90 : 10.

13. The method according to Claim 8, wherein the solvent dissolving entecavir is at least one of dimethyl sulfoxide, dimethylformamide, acetic acid, hydrochloric acid, methanol, ethanol and water for injection.

14. The method according to Claim 13, wherein the solvent is at least one of dimethylformamide, acetic acid and hydrochloric acid.

15. A pharmaceutical composition for parenteral administration comprising an entecavir microsphere as defined in Claim 1 and a pharmaceutically acceptable carrier.

16. The pharmaceutical composition according to Claim 15, which is an injection.
